# EUROPEAN PATENT SPECIFICATION

(11) **EP 3 071 272 B1**
(45) Date of publication and mention of the grant of the patent: **09.01.2019**
(21) Application number: 14809955.9
(22) Date of filing: 18.11.2014
(51) Int. Cl.: A61M 15/00

(54) **INHALER DEVICE**
INHALATOR
DISPOSITIF INHALATEUR

(30) Priority: 19.11.2013 IN 3634MU2013
(43) Date of publication of application: 28.09.2016
(73) Proprietor: Cipla Limited, Mumbai 400 013 (IN)
(72) Inventor: MALHOTRA, Geena, 400 010 Mumbai (IN); RAO, Xerxes, 400052 Mumbai (IN); MHAPSEKAR, Vrinda, 400 101 Mumbai (IN)
(74) Representative: Bone, Alexander Marcus Thomas
(86) International application number: PCT/GB2014/053405
(87) International publication number: WO 2015/075433

(56) References cited:
- EP-A2- 0 806 214
- WO-A2-2008/042951
- GB-A- 2 485 858
- US-A1- 2009 320 838

## Description

### Field of Invention:

The present invention relates to an inhaler/inhalation device for facilitating the inhalation of medicaments.

### Background & Prior Art:

Dry powder inhalation (DPI) devices are generally used for inhalation of powdered medicament from capsules. These DPI devices may dispense either a single dose or multiple doses of medicament. In single dose or multiple dose DPI formulations, the medicament is pre-packaged in capsules (or blisters). Reservoir DPI formulations involve a device containing at least a reservoir and a metering chamber to administer an accurate dose of the medicament.

Medicament holders for inhalation devices known in the art are used for receiving and holding a medicament, which is typically in granular or powder form encapsulated within a capsule (or blister). The configuration of the holder is such that the medicament or the capsule is located inside a chamber and rotated or moved up and down by air flowing through the chamber in order to disperse the drug medicament into air being inhaled by a user of the inhalation device.

The prior art medicament holders generally have an elongate tubular or cylindrical shape with an air inlet and air outlet arranged coaxially at opposite ends thereof. The air inlet is generally smaller and the outlet opening generally larger than the diameter of the capsule. A mouthpiece is arranged axially to the air outlet. Generally, a sprung biased piercing mechanism is provided to pierce the capsule to enable the medicament present in the capsule to be released and inhaled. During inhalation, air passes from the air inlet through the chamber of the holder and causes movement of the capsule. This movement encourages medicament to be released from the capsule and be carried by the air to the air outlet opening and mouthpiece of the inhalation device and onwards to the lungs of the patient (user).

In a single dose DPI device, a single capsule is manually placed in the holder chamber prior to use. Once the capsule has been pierced and the medicament dispensed (inhaled), the empty capsule remains in the device and must be discarded from the device manually prior to the next use of the device.

The following documents disclose known inhalation devices.
US Patent No 3,807,400 discloses an inhaler comprising an upper member comprising a whirling chamber; a lower member comprising a capsule-receiving chamber and a series of cams and recesses on the inner wall of the lower member; and piercing members resiliently biased away from a capsule received in the capsule-receiving chamber by the action of a spring. In use, rotation of upper and lower members relative to each other causes reciprocations of the piercing members against the cams and recesses provided on the inner wall of the lower member, so as to effect a number of diametrically opposed piercing operations on the capsule.

US Patent No 3,795,244 discloses an inhaler comprising a housing; a rotary member located within the housing and having at one end engagement means adapted to receive a medicament-containing capsule; and a piercing assembly comprising opposed arms with piercing pins mounted thereon and a tubular member slidable relative to the housing and having cam projections from the inner wall. In use, and prior to inhalation, the user inserts a capsule into the engagement means and is then required to manually slide the tubular member towards the mouthpiece, so that the cams provided on the inner wall of the tubular member push opposed piercing arms towards the capsule, resulting in perforation of the capsule at its free end. The user is then required to manually return the tubular member to its original position, allowing the piercing arms to return to the non-piercing position by virtue of the normal resilient nature of plastic material of the arms.

US Patent No 3,635,219 discloses a dry powder inhaler device comprising a propeller-like member rotatably mounted in a housing and having mounting means adapted to receive a medicament-containing capsule. In use, flow of inhaled air through the device causes rotation and vibration of the propeller-like member and a capsule mounted thereon, so dispensing medicament into the air stream. The device further comprises spring-loaded piercing members mounted in the housing so as to be normally urged into an inoperative position but which may be manually pushed inwards to perforate a medicament containing-capsule received in the device by the action of pushbuttons or sliding cams.

All of the abovementioned known inhalation devices require the user of the device to open the device, insert a medicament-containing capsule, close the device, and pierce the capsule manually by a pushing or rotating action prior to inhalation of the medicament. The user has to perform numerous actions before actual inhalation of the medicament. Also, the devices rely on a spring arrangement to return a piercing member to an inoperative (non-piercing) position. This use of a spring arrangement can complicate the assembly of the device (for example, due to the need to compress the spring to allow assembly) and also causes the effectiveness with which a piercing member is moved to be dependent upon to the strength of the spring.

Improved inhalers are described in applications WO 2006/051300 and WO 2007/144659. Further improvements over these known devices have now been made.

### Object of the Invention:

An object of the present invention is to provide an inhaler device which provides reduced number of actions before actual inhalation of the medicament

Another object of the present invention is to provide an inhaler device with better improvements of the prior available devices thereby allowing easy operation.

### Summary of the Invention:

According to the present invention, there is provided an inhalation/ inhaler device for facilitating the inhalation of a medicament from a pierceable medicament capsule, the inhaler device comprising a body (1) having a chamber (14) for receiving a pierceable medicament capsule; piercing means (7) for piercing a medicament capsule received in said chamber (14); an actuator (4,4') rotatably mounted to the body (1); and an actuating member (3) moveable relative to the body (1) so as to rotate said actuator (4,4'); characterised by a cam track member (5,5') from which the piercing means (7) extend and comprising a plurality of cam track parts (200,600,200',330') along which said actuator (4,4') slides; wherein a first movement (501,601) of the actuating member (3) relative to the body (1) slides said actuator (4,4') along and in abutment with a first one (200,200') of said cam track parts so as to press against said cam track member (5,5') and thereby drive the piercing means (7) towards an extended position; and wherein a second movement (502,602) of the actuating member (3) relative to the body (1) slides said actuator (4,4') along and in abutment with a second one (600,330') of said cam track parts so as to press against said cam track member (5) and thereby drive the piercing means (7) towards a retracted position.

### Detailed description of the Invention:

As discussed hereinabove, and according to the present invention, there is provided an inhaler device (201) may be provided as mentioned above, wherein said abutment with said first cam track part (200') is by a first cam member (180') of said actuator (4'), and said abutment with said second cam track part (330') is by a second cam member (190') of said actuator (4'). Furthermore, an inhaler device (201) may be provided wherein a third movement (603) of the actuating member (3) relative to the body (1) slides said second cam member (190') of said actuator (4') along and in abutment with a third one (300') of said cam track parts so as to press against said cam track member (5') and thereby drive the piercing means (7) towards a retracted position; and wherein a fourth movement (604) of the actuating member (3) relative to the body (1) slides said first cam member (180') of said actuator (4') along and in abutment with a fourth one (230') of said cam track parts so as to press against said cam track member (5') and thereby drive the piercing means (7) towards the extended position. Ideally, wherein, in this inhaler device (201), said actuating member (3) is moveable back and forth between first and second limits of movement, and wherein, movement of said actuating member (3) from the first limit to the second limit comprises in sequence said first movement (601) followed by said third movement (603) of said actuating member (3), and wherein, movement of said actuating member (3) from the second limit to the first limit comprises in sequence said fourth movement (604) followed by said second movement (602) of said actuating member (3).

Alternatively, the inhaler device (100) of the present invention may be provided wherein said abutment with said first cam track part (200) is by a first cam member (180) of said actuator (4), and said abutment with said second cam track part (600) is by said first cam member (180).

Ideally, an inhaler device (100) is provided wherein said actuator (4) comprises a second cam member (190); and wherein a third movement (503) of the actuating member (3) relative to the body (1) slides said second cam member (190) along and in abutment with a third one (330) of said cam track parts so as to press against said cam track member (5) and drive the piercing means (7) from an extended position to a partially retracted position, and wherein said second movement (502) of the actuating member (3) relative to the body (1) slides said first cam member (180) of said actuator (4) along and in abutment with said second one (600) of said cam track parts so as to press against said cam track member (5) and thereby drive the piercing means (7) from said partially retracted position to a fully retracted position.

An inhaler device (100) may also be provided wherein a fourth movement (504) of the actuating member (3) relative to the body (1) slides said first cam member (180) of said actuator (4) along and in abutment with a fourth one (230) of said cam track parts so as to press against said cam track member (5) and thereby drive the piercing means (7) towards an extended position; and wherein a fifth movement (505) of the actuating member (3) relative to the body (1) slides said first cam member (180) of said actuator (4) along and in abutment with a fifth one (500) of said cam track parts so as to press against said cam track member (5) and thereby drive the piercing means (7) towards the fully retracted position; and wherein a sixth movement (506) of the actuating member (3) relative to the body (1) slides said second cam member (190) along and in abutment with a sixth one (300) of said cam track parts so as to press against said cam track member (5) and drive the piercing means (7) from an extended position to said partially retracted position, and wherein said fifth movement (505) of the actuating member (3) drives the piercing means (7) from said partially retracted position to a fully retracted position.

An inhaler device, according to the present invention, wherein said actuating member (3) is moveable back and forth between first and second limits of movement, and wherein, movement of said actuating member (3) from the first limit to the second limit comprises in sequence said first movement (501) followed by said sixth movement (506) followed by said fifth movement (505) of said actuating member (3), and wherein, movement of said actuating member (3) from the second limit to the first limit comprises in sequence said fourth movement (504) followed by said third movement (503) followed by said second movement (502) of said actuating member (3).

It is preferable for an inhaler device to be provided, wherein, at said first limit of movement, the chamber (14) is closed by said actuating member (3) so as to retain captive in the chamber (14) a medicament capsule received in said chamber (14), and wherein, at said second limit of movement, the chamber (14) is open so as to allow release from the chamber (14) a medicament capsule received in said chamber (14).

Preferably, said body (1) comprises means constraining movement of said cam track member (5) to reciprocal linear movement within a single plane relative to the body (1). Ideally, said plurality of cam track parts (200,600,200',330') are located in said single plane. Also, said actuator (4) may be rotatable about an axis extending perpendicularly to said single plane. It is also preferable for the or each cam member (180) of said actuator is located in said single plane.

Furthermore, the piercing means (7) is ideally located in said single plane. Also, said constraining means preferably abuts said cam track member (5) so as to constrain movement thereof.

Additionally, said actuating member (3) may comprise a mouthpiece through which, in use, a medicament from a pierceable medicament capsule in the chamber (14) is inhaled. Also, it is preferable that, in the extended position, at least a part of the piercing means (7) extends within said chamber (14) from one side of said chamber (14) towards an opposite side thereof.

It is more preferable for the inhaler to comprise means (800) of providing a point contact to a medicament capsule located in the chamber (14).

Embodiments of the present invention will now be described with reference to the accompanying drawings (which in no way restrict the scope of the invention and are for the purpose of illustration only), in which:
Figure 1 is a perspective view of a first inhalation device according to the present invention, with piercing means (not visible) in a substantially extended position and the inhaler mouthpiece in a partly opened position;
Figure 2 is a perspective view of the inhalation device of Figure 1, with piercing means (not visible) in a retracted position and the inhaler mouthpiece in a closed position;
Figure 3 is a side view of the inhalation device of Figure 1, with piercing means (not visible) in a retracted position and the inhaler mouthpiece in a closed position;
Figure 4 is a side view of the inhalation device of Figure 1, with piercing means (not visible) in a retracted position and the inhaler mouthpiece in an opened position;
Figure 5 is an exploded view of the parts of the inhalation device of Figure 1;
Figure 6a is an internal side view of the inhalation device of Figure 1, wherein an actuator is located in a position corresponding to the piercing means being fully retracted and the inhaler mouthpiece being in a fully opened position;
Figure 6b is an internal side view of the inhalation device of Figure 1, wherein the actuator is located in a position corresponding to the piercing means being fully extended and the inhaler mouthpiece being in a partially opened position;
Figure 6c is an internal side view of the inhalation device of Figure 1, wherein the actuator is located in a position corresponding to the piercing means being fully retracted and the inhaler mouthpiece being in a fully closed position;
Figure 7 is an internal perspective view of the interior of a lower body of the inhalation device of Figure 1, wherein the actuator and piercing means are shown;
Figure 8 is a cross-sectional side view of a second inhalation device according to the present invention, with piercing means in a retracted position and the inhaler mouthpiece in a closed position;
Figure 9 is a cross-sectional side view of both first and second inhalation devices, with piercing means thereof in a retracted position and the inhaler mouthpieces in a closed position (a first limit of movement of the mouthpiece corresponding to zero degrees);
Figure 10 is a cross-sectional side view of both first and second inhalation devices, with piercing means thereof in a partially extended position and the inhaler mouthpieces in a partially opened position (the mouthpieces having moved from a zero degree position through an angle of twenty five degrees);
Figure 11 is a cross-sectional side view of both first and second inhalation devices, with piercing means thereof in a fully extended position and the inhaler mouthpieces being mid-way between fully closed and fully opened positions (the mouthpieces having moved from a zero degree position through an angle of fifty degrees);
Figure 12 is a cross-sectional side view of both first and second inhalation devices, with piercing means thereof in a partially retracted position and the inhaler mouthpieces being in a partially opened position (the mouthpieces having moved from a zero degree position through an angle of seventy five degrees);
Figure 13 is a cross-sectional side view of both first and second inhalation devices, with piercing means thereof in a partially retracted position and the inhaler mouthpieces being in a partially opened position (the mouthpieces having moved from a zero degree position through an angle of eighty seven degrees);
Figure 14 is a cross-sectional side view of both first and second inhalation devices, with piercing means thereof in a partially retracted position and the inhaler mouthpieces being in a partially opened position (the mouthpieces having moved from a zero degree position through an angle of ninety degrees);
Figure 15 is a cross-sectional side view of both first and second inhalation devices, with piercing means thereof in a partially retracted position and the inhaler mouthpieces being in a partially opened position (the mouthpieces having moved from a zero degree position through an angle of ninety five degrees);
Figure 16 is a cross-sectional side view of both first and second inhalation devices, with piercing means thereof in a retracted position and the inhaler mouthpieces in a fully opened position (a second limit of movement of the mouthpiece corresponding to one hundred degrees);
Figure 17 is a side view of a cam track member 5 of said first inhaler with six phases of movement of the mouthpiece in moving from the closed to open and back to closed positions being illustrated; and
Figure 18 is a side view of a cam track member 5' of said second inhaler with four phases of movement of the mouthpiece in moving from the closed to open and back to closed positions being illustrated.

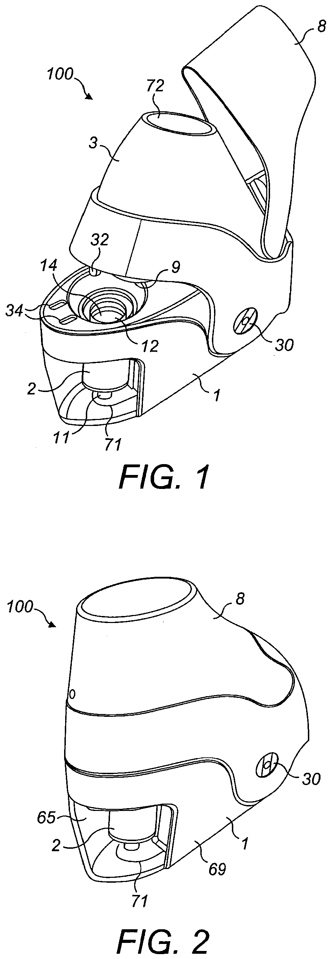

A first embodiment 100 of an inhalation device according to the present invention is shown in Figures 1 to 7 of the accompanying drawings.

The inhalation device 100 comprises a lower body 1 and a mouthpiece 3 attached to the lower body by a pivot 30 about which the mouthpiece 3 is rotatable relative to the lower body 1. The pivot 30 projects laterally from an actuator 4 of the lower body 1 and serves to connect the mouthpiece 3 to the lower body 1, and more particularly, to the actuator 4.

The lower body 1 includes a number of internal parts and an outer shell which houses said internal parts.

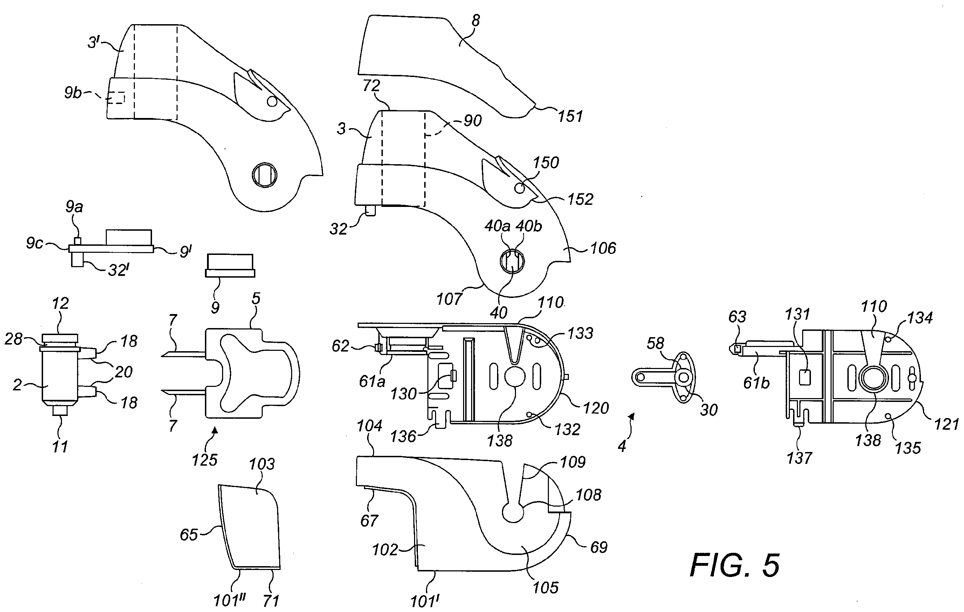

The outer shell of the lower body 1 is most clearly shown in Figure 5 as including a primary shell piece 69 and a discrete and transparent end shell piece 65. The primary shell piece 69 has a conventional clip feature 67 integral therewith which either snap-fits or push-fits into the end shell piece 65 and thereby retains the two pieces 65,69 in abutment with one another to form said outer shell. The transparent end shell piece 65 acts as a window and thereby allows a capsule holder 2 to be viewed by a user.

The primary shell piece 69 has a bottom surface 101' and two side walls 102 (which are mirror images of one another, but only one of which is visible in the accompanying drawings) upstanding from the bottom surface 101'. The end shell piece 65 also has a bottom surface 101" (which adjoins the bottom surface 101' of the primary shell piece 69 to form a bottom surface 101 of the assembled outer shell) and a curved end wall 103 (which adjoins with the two side walls 102 of the primary shell piece 69 in the assembled outer shell). The combined shape of the two pieces is such that the assembled outer shell forms a cup-like container (for housing said internal parts) with an opening 104 in the top thereof.

A primary air (inhalation air) inlet 71 of the inhalation device 100 is provided in the bottom surface 101" of the end shell piece 65. The inlet 71 may be of any suitable shape, for example, an oval shape.

A recess 105 is provided in a part of each wall 102 of the primary shell piece 69. The recess 105 has a shape (side profile, as seen in Figure 5 for one wall) and depth such that, in the assembled inhalation device 100, two legs 106 (only one of which is visible in the accompanying drawings) of mouthpiece 3 snap-fit on the actuator 4 and locate either side of the outer shell and in the recesses 105 so that, in a region adjacent the end shell piece 65, there is substantially no step between the outer shell and the mouthpiece when the mouthpiece is in a closed position (see Figure 2 in particular) - in other words, the outer surfaces of outer shell and the mouthpiece are substantially flush with one another. This provides the inhaler device with a smooth outer surface and a desirable aesthetic quality as a result.

It is emphasised that the recess 105 in each wall 102 does not prevent the mouthpiece 3 from moving between opened and closed positions. This is because the walls 102 of the primary shell piece 69 are positioned progressively closer to one another (i.e. they converge) when moving from the end shell piece 65 towards the pivot 30 and beyond. As such, a part-circular lower edge 107 of each leg 106 (which edge 107 is concentric with the pivot 30) maintains a position substantially spaced from the primary shell piece 69 when the mouthpiece 3 is moved between opened and closed positions.

The primary shell piece 69 is also provided with a circular aperture 108 extending through the full thickness of each wall 102 of the primary shell piece 69. The circular aperture 108 of one wall 102 aligns with the aperture 108 of the other wall 102 and, in the assembled lower body 1, the pivot 30 extends through each aperture 108 so as to project laterally/outwardly from each wall 102 and receive (in a snap-fitting relationship therewith, as alluded to above) the two legs 106 of the mouthpiece 3 (said legs thereby snap-fitting on the actuator 4).

Furthermore, the primary shell piece 69 is also provided with a V-shaped aperture 109 extending through the full thickness of each wall 102. The converging walls of the V-shaped aperture 109 converge from the opening 104 in the top of the outer shell towards, and terminate at, the aforementioned circular aperture 108. The purpose of the V-shaped aperture is to prevent the pivot 30 from hindering movement of said internal parts into the outer shell. In this respect, during assembly of the inhalation device 100, said internal parts are themselves assembled and then moved (from the position above the primary shell piece 69 shown in Figure 5) through said opening 104 of the outer shell and into the interior of the outer shell. In moving said internal parts into the outer shell, the pivot 30 (provided on the actuator 4) moves through the V-shaped apertures 109. The distance between opposite walls of each V-shaped aperture 109 at the point where they terminate at their respective circular apertures 108 has a magnitude less than that of the diameter of the pivot 30. The diameter of each circular aperture 108 is the same as or larger than the diameter of the pivot 30, the relative sizes of said apertures 108 and the pivot 30 being such as to allow the pivot 30 to rotate within the apertures 108 without substantial resistance. However, the relative sizes of said V-shaped apertures 109 and the pivot 30 is such that movement of the pivot 30 along the V-shaped apertures towards the circular apertures 108 is resisted, but not prevented, such that the pivot 30 snap-fits into the circular apertures 108 during assembly of the lower body 1. In the assembled lower body 1, first and second V-shaped projections 110, extending laterally from either side of the assembled internal parts, fill the V-shaped apertures 109 and preserve the aesthetic quality of the inhalation device 100, particularly when in an open configuration (i.e. with the mouthpiece 3 in a opened position).

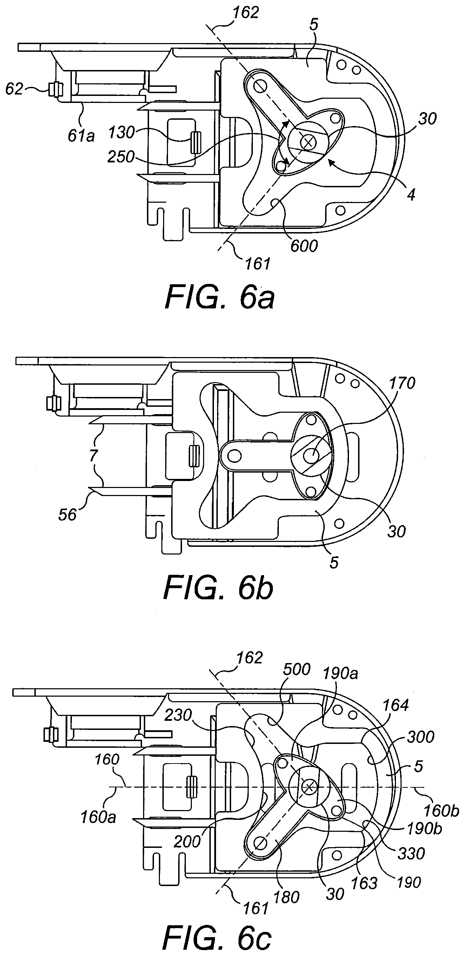

The internal parts of the lower body 1 are shown most clearly in Figures 5 to 7. It is to be noted however that one of said internal parts, namely a capsule holder 2, is not shown in Figures 6 to 7, and a further internal part (one of two cooperating actuator case members 121 - see below) is not shown in Figures 6a, 6b and 6c for the purposes of clarity.

The principal objectives of the internal parts are to hold a medicament capsule in place and to pierce said capsule when the mouthpiece 3 is moved, and specifically, in the present embodiment, when the mouthpiece 3 is moved from an opened position to a closed position relative to the lower body 1.

Detail regarding said internal parts is provided below.
Firstly, the internal parts of the lower body 1 include a capsule holder 2. This holder 2 is a discrete and transparent part and comprises a chamber 14 adapted to receive a medicament-containing capsule, such as a dry powder medicament-containing capsule. In use of the inhalation device 100, a capsule is placed into the chamber 14, pierced and discharged of its contents whilst remaining in the chamber 14, and then removed from the chamber 14.

The chamber 14 is cylindrical in shape and has an air inlet 11 and an air outlet 12. It will be understood by those skilled in the art that, in a different embodiment, the chamber 14 may be an alternative shape, such as conical. The air inlet 11 and air outlet 12 are located at opposite axial ends of the chamber 14. The air inlet 11 extends through a boss which itself extends downwardly from the bottom of the chamber 14. A wall of the chamber 14 is provided with two openings 18 which are axially spaced from one another along the length of the cylindrical chamber 14 so as to each receive a different one of two piercing pins 7.

The holder 2 further comprises guide means 20 which are provided integrally with the chamber 14, are generally cylindrical in shape, and extend laterally from the openings 18 to the exterior of the chamber 14. The arrangement is such that said means 20 guide a back and forth movement of the piercing pins 7 into and out of the chamber 14 in response to a rotation of the mouthpiece 3 about the pivot 30. Support is thereby provided for the piercing pins 7 at all locations of the pins 7 at and between fully extended and fully retracted positions. The guide means 20 can be best appreciated from Figure 5 of the accompanying drawings.

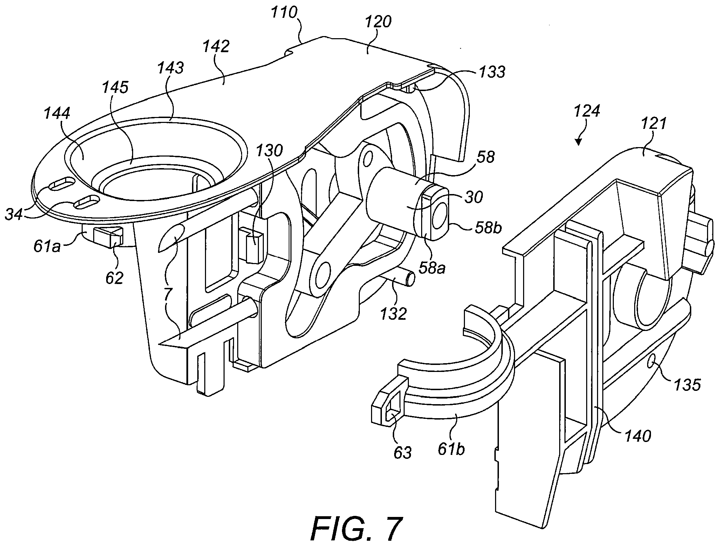

The capsule holder 2 is yet further provided with an annular groove 28 at an upper end thereof, adjacent the outlet 12. This groove 28 enables the holder 2 to be secured to the remainder of said internal parts. Specifically, a two-piece locating ring 61a,61b is provided for locating around the upper end of the holder 2 and in the groove 28 (see Figures 5 and 7). The two-pieces 61a,61b of said ring snap-fit together by means of a resiliently and elastically deformable clip member 62 which projects from a first 61a of the ring pieces and snap-fits into an aperture 63 provided in a second 61b of the ring pieces.

The aforementioned internal parts of the lower body 1 further include first and second cooperating actuator case members 120,121 which snap-fit and press-fit to one another to form an actuator case. The assembled actuator case 120,121 serves a number of functions, as described below, but principally, the assembled actuator case 120,121 provides a structure for supporting the remaining internal parts and for securing said remaining internal parts in the outer shell of the lower body 1.

More specifically, the first case member 120 is integrally formed with the first ring piece 61a and the second case member 121 is integrally formed with the second ring piece 61b. In the assembled actuator case, the aforementioned two-piece locating ring 61a,61b is provided cantilevered from the remainder of the actuator case. The capsule holder 2 is suspended from the ring 61a,61b. Also, in the assembled actuator case 120,121, a cavity 124 (see Figure 7) is provided between the first and second cooperating case members 120,121 in which the actuator 4 and capsule piercing means 125 are located. The arrangement (i.e. the relative size and shape) of the capsule piercing means 125 and the actuator case 120,121, in particularly the cavity 124, is such that the piercing means 125 is constrained by the actuator case 120,121 to move linearly back and forth, towards and away from the capsule holder 2, without any substantial rotational movement in any plane relative to the actuator case 120,121.

The capsule piercing means 125 includes a cam track member 5 and the aforementioned two piercing pins 7 which extended from the cam track member 5, parallel to one another and to the direction of linear movement of the piercing means 125, into the aforementioned guide means 20. The guide means 20 also contribute to constraining movement of the piercing means 125 to linear movement without rotational movement.

In the devices shown, at least the capsule piercing means 125, the actuator case 120,121 and the guide means 20 combine to form a means for constraining movement of the cam track member 5, 5' to reciprocal linear movement within a single plane relative to the body 1. It should be understood that the means for constraining movement should not be limited by these examples and may comprise one, some, all or none of these components, and may comprise one or more additional components or modifications which co-operate to constrain movement of the cam track member.

Each pin 7 is a discrete solid cylinder with a piercing end thereof comprising a face 56 formed by an oblique cut through the shaft of the pin 7 (see Figure 6b). Modification of the piercing end may be made to achieve a desired shaped point. The pins 7 may also be of any suitable size. They may be in a range between 1 mm to 2 mm in diameter, although the pins 7 of the present embodiment have a diameter of 1.5mm +/- 10%.

The cam track member 5 comprises a closed loop of material lying in a single plane. Although provided as a closed loop, it will be understood that the cam track member can comprise a discontinuity so as not to be a closed loop. The piercing pins 7 lie in the same plane. The internal surface of the closed loop is precisely shaped to define a cam track against which cams of the actuator 4 press so as to move the cam track member 5 (and hence the piercing pins 7) as the mouthpiece 3 is moved. As shown in Figures 6 and 7, when the internal parts of the lower body 1 are assembled, the actuator 4 is position within the closed loop of the cam track member 5. In this way, the cams of the actuator 4 may be located in the same plane as the piercing pins 7 and cam track member 5, and said cams can then press on the cam track.

Further detail of the cam track and its operation in relation to the actuator 4 will be provided below. Prior to this further detail, the remaining features of the actuator case 120,121 will be described.

Firstly, the first and second case members 120,121 include snap-fit and press-fit features for securing the two members 120,121 together. In this regard, and as previously mentioned, a resiliently and elastically deformable clip member 62 projects from the first ring piece 61a of the first case member 120 and snap-fits into an aperture 63 provided in the second ring piece 61b of the second case member 121. Similarly, a resiliently and elastically deformable clip member 130 projects from the first case member 120 at a location between the two piercing pins 7 and snap-fits into an aperture 131 provided in the second case member 121 (see Figure 5). Also, two bosses 132,133 projecting from the first case member 120 locate with a press-fit into cooperating apertures 134,135 in the second case member 121 (see Figure 5).

Furthermore, a resiliently and elastically deformable clip member 136 projects downwardly from a lower edge of the first case member 120 and, in the assembled actuator case, locates adjacent a similar resiliently and elastically deformable clip member 137 projecting downwardly from a lower edge of the second case member 121. In the assembled lower body 1, the two clip members 136,137 snap-fit into an aperture (not shown) provided in the bottom surface 101' of the primary shell piece 69. This not only assists in securing the two case members 120,121 together, but also assists in retaining the assembled internal parts in the outer shell of the lower body 1.

Each of the two case members 120,121 is provided with a different one of the two V-shaped projections 110. Each of the two case members 120,121 is also provided with a circular aperture 138 located adjacent an apex of the V-shaped projection 110 of the case member and extending through the full thickness of the case member. In the assembled actuator case, the pivot 30 of the actuator 4 extends through the aperture 138 of each case member 120,121. The diameter of each circular aperture 138 is the same as or larger than the diameter of the pivot 30, the relative sizes of said apertures 138 and the pivot 30 being such as to allow the pivot 30 to rotate within the apertures 138 without substantial resistance.

In addition, each of the two case members 120,121 is provided with a straight external groove 140 (see Figure 7). Each groove 140 receives a projection (not shown) extending from a different one of the internal surfaces (not shown) of the two side walls 102 of the primary shell piece 69. The grooves 140 are aligned with the direction in which the assembled internal parts are moved into the assembled outer shell and thereby assist with a correct location of the internal parts within the assembled outer shell.

Finally, the first case member 120 includes a top surface 142 which is generally perpendicular to the plane in which the pins 7 are located, and which, in the assembled lower body 1, closes the opening 104 in the assembled outer shell of said body 1.

An aperture 143 is provided in the top surface 142 with a frusto-conical shaped fluid passageway 144 extending downwards beneath said top surface 142 from said aperture 143 and terminates with an annular collar 145 (see Figure 7). The annular collar 145 provides a step reduction in the internal diameter of the fluid passageway 144. The annular collar 145 lies in a plane parallel to the top surface 142. The fluid passageway converges in a downward direction away from the top surface 142. When the internal parts are assembled, the annular collar 145 locates above and in abutment with the air outlet 12 of the chamber 14 and the fluid passageway 144 is thereby connected to the air outlet 12 of the chamber 14.

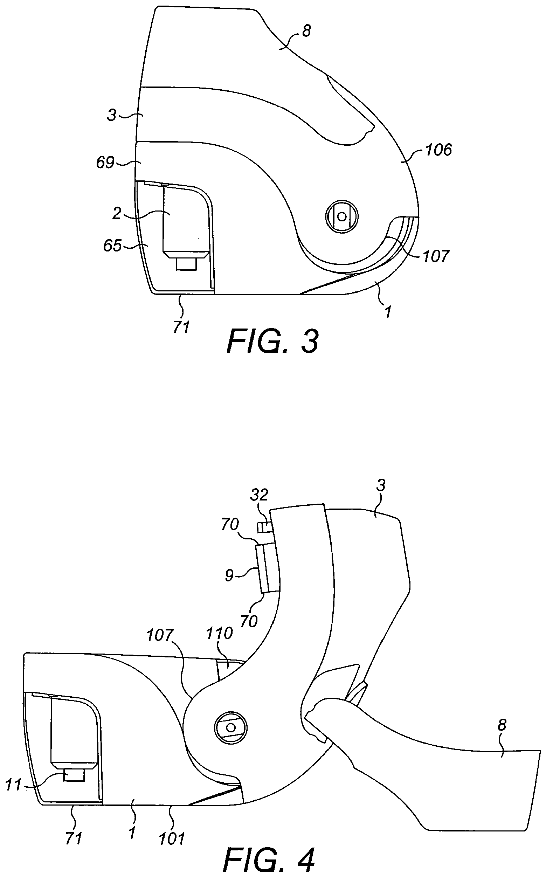

The preferred position (denoted by the reference numeral 70) of optional auxiliary vents (inlets) is shown in Figure 4. The auxiliary vents allow a flow of inhalation air into the mouthpiece in addition to that entering the inhaler via the primary inlet 71. The vents themselves are not shown in the drawings relating to the inhalation device 100 of Figures 1 to 7. As suggested, the auxiliary vents are optional, and may be placed at any position in the inhalation passage, although it is preferred to position them upstream of medicament capsule.

The outer diameter of the annular collar 145 is greater than the outer diameter of the portion of chamber 14 with which the collar 145 abuts. In this way, holes may be optionally provided through the collar 145 so as to provide the aforementioned auxiliary vents and thereby allow air (located outside the chamber 14 and beneath the collar 145) to flow into the fluid passageway. The vent holes may have a diameter of up to 1 mm. Six vent holes may be provided. The vent holes may be spaced equidistant from one another. Alternatively, two crescent-shaped auxiliary vents may be provided in the collar 145, located opposite each other.

Furthermore, when the mouthpiece is in the closed position in the assembled inhalation device 100, the fluid passageway 144 also connects to a fluid passageway in said mouthpiece 3. A continuous fluid pathway from the interior of the chamber 14 to an outlet of the mouthpiece 3 is thereby provided.

The top surface 142 is also provided with two holes 34 located on a side of the aperture 143 opposite the pivot 30. The two holes 34, together with two resiliently and elastically deformable projections 32 extending from the mouthpiece 3, provide latching means for latching the mouthpiece 3 in a predetermined position relative to the lower body 1, for example in the fully closed position. Each projection 32 is adapted to be received by a different one of the two holes 34. The projections 32 locate in their respective holes 34 with a snap-fit and cooperate with said holes 34, when the mouthpiece 3 is closed, and serve to provide resistance to the mouthpiece 3 being moved from the closed position. The mouthpiece 3 can thus be secured in the fully closed position. The resistance of the projections 32 snap-fitted in their respective holes 34 can nevertheless be readily overcome by the user so that the mouthpiece 3 can be moved from the closed position towards the opened position. In an alternative embodiment, the projections 32 may instead be provided in another element of the inhalation device, for example as an integral part of the mesh unit 9 which is located in the mouthpiece 3. Other commonly used methods of retaining the mouthpiece in the closed position non-permanently can also be used.

In addition to the lower body 1, the inhalation device 100 includes a closure means for closing the capsule chamber 14 and thereby preventing a capsule from falling from the chamber 14 or being sucked therefrom by a user. The closure means is the mouthpiece 3 in the present embodiment 100 of the invention.

In an embodiment of the invention, the closure means may function as an actuating member, which moves the actuator 4. This is the arrangement shown in the accompanying drawings. In an alternative embodiment, the closure means may be a component other than a mouthpiece. Furthermore, in another embodiment of the invention, an actuating member may be provided which is neither a closure means nor a mouthpiece.

The mouthpiece 3 includes a cover 8 (for closing an opening (air outlet) 72 of the mouthpiece 3 when not in use) and a discrete mesh unit 9 (for filtering inhalation air during use).

The mouthpiece 3 has two legs 106 (only one of which is shown in the accompanying drawings) extending from the sides thereof. Each leg 106 has an aperture 40 therein, with each aperture having opposing flat surfaces 40a,40b which enable the mouthpiece 3 to connect to an opposing pair of flats 58a,58b (i.e. flat surfaces) provided on a cylindrical portion 58 of the actuator 4. The legs 106 of the mouthpiece 3 are resiliently and elastically deformable, and, in the assembled device, snap-fit onto either side of the lower body 1, specifically with each aperture 40 locating about a different opposite end of the cylindrical portion 58 of the actuator 4. A torque associated with a rotary movement of the mouthpiece 3 relative to the lower body 1 is transmitted to the actuator 4 by abutment of the flat surface denoted by reference numeral 40a with the flat surface denoted by reference numeral 58a, and similarly, by abutment of the flat surface denoted by reference numeral 40b with the flat surface denoted by reference numeral 58b. Rotary movement of the actuator 4 results in a linear movement of the piercing means 125, as will be described in greater detail below.

As mentioned above, the mouthpiece 3 comprises an opening (air outlet) 72 through which medicament is inhaled by the user. Means for receiving air from an outlet 12 of the lower body 1 extends downwards from the opening 72 within the mouthpiece. In the present embodiment, said means for receiving air is a tube 90.

The aforementioned mouthpiece cover 8 is rotationally attached to the remainder of the mouthpiece 3 by pivot means 150. The cover 8 is rotatable relative to the mouthpiece 3 between a closed position, in which the cover 8 closes the air outlet 72, and an open position, in which the cover 8 is spaced from the air outlet 72 so as to allow a user to place his or her mouth about the outlet 72 and inhale medicament in the chamber 14. The cover 8 is retained in the closed position and in the open position by suitable means, for example by an over-centre spring bias arrangement. In the present embodiments, the over-centre spring bias arrangement comprises a projection 151 on either side of the cover 8, adjacent the pivot means 150, which presses on an associated cooperating projection 152 on the remainder of the mouthpiece 3. As the cover 8 moves from the open position or from the closed position and a projection 151 on the cover 8 presses on the projection 152, rotation of the cover 8 is thereby resisted and the cover 8 tends to be biased back towards its original position. This resistance is sustained until the projection 151 on the cover 8 rides over and passed the projection 152 on the remainder of the mouthpiece 3.

The means for retaining the cover 8 in the closed position presents less resistance to movement than is provided by the projections 32, so that, when a user applies an opening force to the cover 8 in an attempt to open the mouthpiece, the cover 8 tends to be moved relative to the opening 72, rather than the mouthpiece (including the cover 8) being moved towards the opened position.

The aforementioned mesh unit 9 (see Figures 1 and 5) is positioned in the mouthpiece 3 so as to align with the aperture 143 in the top surface 142 of the lower body 1 when the mouthpiece 3 is in the fully closed position. When the mouthpiece 3 is in the closed position (as shown in Figures 2 and 3) and the mesh unit 9 is positioned over said top surface aperture 143 and said chamber outlet 12, the mesh unit 9 retains a capsule received in the chamber 14, preventing the capsule from falling out of the inhalation device 100, or being sucked into the outlet/mouthpiece upon inhalation by the user. Alternatively, as shown in Figure 5, projections 32' can be provided as a part of an alternative mesh unit 9' to retain the mouthpiece in the closed position as described above. When the projections 32' are provided as part of the mesh unit 9', the mesh unit may further comprise a planar element 9c projecting from the mesh unit 9'. The projections 32' may be provided on a lower surface of the planar element 9c such that the projections 32' engage the aforementioned holes 34 when the mouthpiece is moved to the closed position. The planar element may be provided with a snap-fit component 9a on its upper surface that engages a complimentary element 9b provided on an alternative mouthpiece 3' in order to secure the mesh unit 9' and projections 32' in place on the mouthpiece 3'. The retaining force of the snap-fit component to the mouthpiece 3' is preferably greater than the retaining force of the projections 32' to the holes 34. The snap-fit component 9a may take the form of a resiliently deformable jaw that grips a complimentary element 9b, such as a ridge, provided on the mouthpiece 3'.

The mesh of the mesh unit 9 can be of any suitable size but is preferably in the order of 0.5mm X 0.5mm square to 2mm X 2mm square. More preferably, the mesh size is 1mm X 1mm square +/- 10%.

When the mouthpiece 3 is in the closed position (as in Figures 2 and 3), the inhalation device 100 defines an inhalation passage extending through the capsule chamber 14 between inlet 71 and a final air (inhalation air) outlet 72 (located in the mouthpiece 3). Referring particularly to Figures 3, 4, 6a, 6b and 6c, during use of the inhalation device 100, the mouthpiece 3 is rotated to a fully opened position in order to open the inhaler 100 (and specifically the chamber 14) and allow a capsule to be readily inserted into the lower body 1 (and, specifically, into the chamber 14). The mouthpiece 3 is then rotated to a fully closed position to close the chamber 14 and position the mouthpiece correctly for inhalation of medicament from the inhaler device.

Rotation of the mouthpiece 3 causes rotation of the actuator 4, which rotation in turn drives movement of the capsule piercing means 125 comprising the cam track member 5 and piercing pins 7.

When the mouthpiece 3 is in either the fully closed or fully opened position, the piercing pins 7 are in a fully retracted, non-piercing position. In other words, the piercing pins 7 do not extend into the chamber 14 so as to hinder the positioning of a medicament capsule therein. In the present embodiment, the piercing pins 7 are entirely removed from the interior of the chamber 14 and are supported (and guided) when in this position by the guide means 20.

When the mouthpiece 3 is partially opened/partially closed, in a position midway between closed (i.e. fully closed) and opened (i.e. fully opened) positions, the piercing pins 7 are located in a fully extended piercing position in which they extend into the chamber 14 from one side thereof to the other. A partially opened position or a partially closed position is a position between, but not at either of, the fully closed and fully opened positions (at first and second limits of movement of the mouthpiece 3 respectively). Similarly, a partially extended position or a partially retracted position is a position between, but not at either of, the extended (i.e. fully extended) and retracted (i.e. fully retracted) positions.

Movement of the mouthpiece 3 from a fully opened to a fully closed position causes movement of the piercing pins 7 from a fully retracted position (when the mouthpiece 3 is in the fully opened position) to a fully extended piercing position (when the mouthpiece 3 is in a position substantially midway between fully closed and fully opened) and back to a fully retracted position (when the mouthpiece 3 is in the fully closed position). Similarly, movement of the mouthpiece 3 from a substantially fully closed position to a substantially fully opened position causes movement of the piercing pins 7 from a fully retracted position to a fully extended piercing position (when the mouthpiece 3 is in a partially opened/partially closed position) and back to the fully retracted position from the fully extended piercing position. Although in a preferred embodiment the pins are fully extended when the mouthpiece 3 is about midway between the opened and closed positions, it will be understood that the fully extended position of the pins may be achieved when the mouthpiece 3 is at any position between the opened and closed positions, depending on the precise design of the device, in particular, of the cam track member 5 and actuator 4.

The arrangement of the cam track member 5 and the actuator 4, and the way in which they cooperate to achieve the abovementioned movement of the piercing means will now be described. In this regard, particular reference should be had to Figures 6a, 6b and 6c. In the following description, the cam track member 5 is considered to comprise six cam track parts - a first track part 200, a second track part 600, a third track part 330, a fourth track part 230, a fifth track part 500, and a sixth track part 300 as shown in Figure 6.

The actuator 4 includes a cylindrical portion 58 which acts as a rotary shaft and cooperates with and extends through the circular apertures 138 of the case members 120,121 and circular apertures 108 of the outer shell. Two cam members 180,190 extend from the cylindrical portion 58 and lie in the plane in which the piercing pins 7 are located. The cam members 180,190 are arranged so as to abut the internal surface of the closed loop of the cam track member 5. This internal surface of the closed loop is precisely shaped to define a cam track against which cam members 180,190 slide and press the cam track member 5. Different cam members 180,190 abut and press against different parts of the cam track.

In this latter regard, first and fourth track parts 200,230 are arranged for abutment with the first cam member 180, and sixth and third track parts 300,330 are arranged for abutment with the second cam member 190. The second cam member 190 can be considered to include two sub cam members 190a,190b. The first sub cam member 190a abuts and presses the sixth track part 300, and the second sub cam member 190b abuts and presses the third track part 330.

With reference to Figure 6c of the accompanying drawings, it will be seen that the cam track is symmetrical about a broken line 160, which line 160 is coincident with the axis 170 of rotation of the cylindrical portion 58 and parallel with both the piercing pins 7 and the constrained direction of linear travel/movement of the cam track member 5.

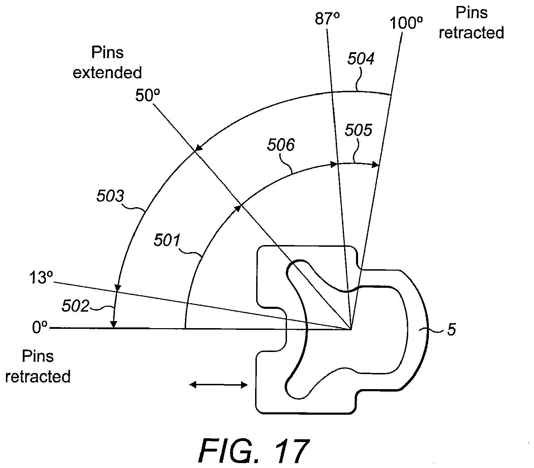

Also, with reference to Figures 6a, 6b and 6c of the accompanying drawings, it will be seen that the mouthpiece 3 and actuator 4 rotate through an angle 250 of 100 degrees in moving between opposite extremes/limits of movement (i.e. between a position corresponding to the mouthpiece 3 being in a closed position, as shown in Figure 6c, and a position corresponding to the mouthpiece 3 being in an opened position, as shown in Figure 6a). In moving from the first limit of movement to the second limit of movement and back to the first limit of movement, there may be considered to be six movements 501,502,503,504,505,506 of the mouthpiece (as shown in Figure 17).

The first track part 200 is that part of the cam track located between broken lines 161 and 160a. As the mouthpiece 3 moves from the fully closed position to a position midway between the closed and opened positions, the actuator 4 is moved from the position shown in Figure 6c as a result, so that the first cam member 180 abuts and presses against the first track part 200, linearly displacing/moving the piercing cam track member 5 in a first direction. As a consequence, the piercing pins 7 are moved from the retracted position to the extended position, wherein the actuator 4 is moved to the position shown in Figure 6b. The second cam member 190 does not abut the sixth and third track parts 300,330 during this phase of movement. It will be appreciated that as the actuator 4 moves in a rotary fashion and the track member 5 is constrained to move in a linear fashion and, as such, during the above phase of movement, the actuator 4 moves along the first track part 200 in sliding abutment therewith. In the above phase of movement, the mouthpiece 3 rotates through an arc of 50 degrees in a first movement 501 of the mouthpiece 3 shown in Figure 17.

The sixth track part 300 is that part of the cam track located between broken line 160b and point 164 shown in Figure 6c. As the mouthpiece 3 moves towards the fully opened position from a position midway between the closed and opened positions, the actuator 4 is moved from the position shown in Figure 6b as a result, so that the first sub cam member 190a abuts and presses against the sixth track part 300, linearly displacing/moving the piercing cam track member 5 in an opposite direction to said first direction. As a consequence, the piercing pins 7 are moved from the extended position towards the retracted position, wherein the actuator 4 is moved towards, although not all the way to, the position shown in Figure 6a. In the above phase of movement, the mouthpiece 3 rotates through an arc of 37 degrees in a sixth movement 506 of the mouthpiece 3 shown in Figure 17.

It will be seen that in the position shown in Figure 6a, a space is present between the second cam member 190 and the sixth and third track parts 300,330. The cam track member 5 is moved in said opposite direction to create this space by the first cam member 180 pressing on a fifth track part 500 (see Figure 6c). The first cam member 180 moves along but does not press against the fourth track part 230 (or first track part 200) during this phase of movement. In the above phase of movement, the mouthpiece 3 rotates through an arc of 13 degrees in a fifth movement 505 of the mouthpiece 3 shown in Figure 17.

It will be appreciated that as the actuator 4 moves in a rotary fashion and the track member 5 is constrained to move in a linear fashion and, as such, during the above phases of movement, the actuator 4 moves along the sixth track part 300 and the fifth track part 500 in sliding abutment therewith.

The fourth track part 230 is that part of the cam track located between broken lines 162 and 160a. As the mouthpiece 3 moves from the fully opened position to a position midway between the closed and opened positions, the actuator 4 is moved from the position shown in Figure 6a as a result, so that the first cam member 180 abuts and presses against the fourth track part 230, linearly displacing/moving the piercing cam track member 5 in said first direction. As a consequence, the piercing pins 7 are moved from the retracted position to the extended position, wherein the actuator 4 is moved to the position shown in Figure 6b. The second cam member 190 does not abut the sixth and third track parts 300,330 during this phase of movement. It will be appreciated that as the actuator 4 moves in a rotary fashion and the track member 5 is constrained to move in a linear fashion and, as such, during the above phase of movement, the actuator 4 moves along the fourth track part 230 in sliding abutment therewith. In the above phase of movement, the mouthpiece 3 rotates through an arc of 50 degrees in a fourth movement 504 of the mouthpiece 3 shown in Figure 17.

The third track part 330 is that part of the cam track located between broken line 160b and point 163 shown in Figure 6c. As the mouthpiece 3 moves towards the fully closed position from a position midway between the closed and opened positions, the actuator 4 is moved from the position shown in Figure 6b as a result, so that the second sub cam member 190b abuts and presses against the third track part 330, linearly displacing/moving the piercing cam track member 5 in an opposite direction to said first direction. As a consequence, the piercing pins 7 are moved from the extended position towards the retracted position, wherein the actuator 4 is moved towards, although not all the way to, the position shown in Figure 6c. In the above phase of movement, the mouthpiece 3 rotates through an arc of 37 degrees in a third movement 503 of the mouthpiece 3 shown in Figure 17.

It will be seen that in the position shown in Figure 6c, a space is present between the second cam member 190 and the sixth and third track parts 300,330. The cam track member 5 is moved in said opposite direction to create this space by the first cam member 180 pressing on a second track part 600 (see Figure 6a). The first cam member 180 moves along but does not press against the first track part 200 (or fourth track part 230) during this phase of movement. In the above phase of movement, the mouthpiece 3 rotates through an arc of 13 degrees in a second movement 502 of the mouthpiece 3 shown in Figure 17.

The actuator 4 moves in a rotary fashion, and the track member 5 is constrained to move in a linear fashion. As such, during the above phase of movement, the actuator 4 moves along the third track part 330 and the second track part 600 in sliding abutment therewith.

The first and fourth track parts 200,230 are arranged relative to the actuator 4 so as to produce a linear movement of the piercing pins 7 of 7 millimeters.

A different movement of the piercing pins 7 can be provided by changing the profile of the cam track (see below).

It will be appreciated that the piercing pins 7 are directly driven by the rotation of the mouthpiece 3, both when extending and retracting the piercing pins. The piercing pins 7 are unlikely therefore to become irreversibly trapped in the extended position when piercing a medicament capsule in the inhaler chamber 14. The actuating/drive arrangement also has a small number of components and links between the mouthpiece 3 and the piercing pins 7, reducing the likelihood of components or links failing.

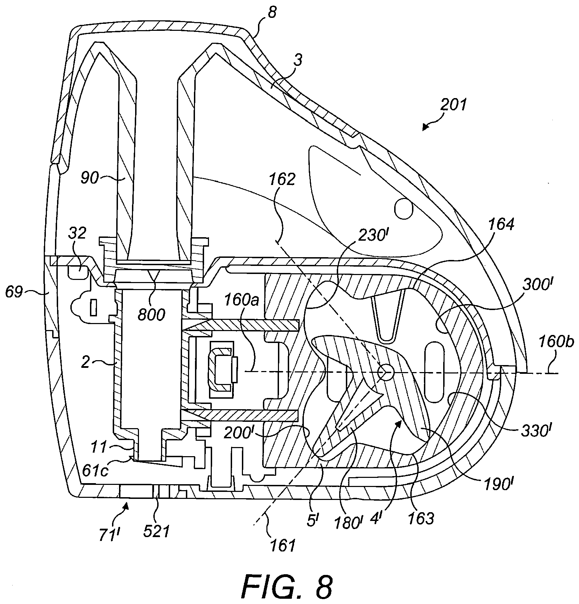

A second inhalation device 201 according to the present invention is shown in Figure 8. The second inhalation device 201 is similar to the first inhalation device 100 in many respects and like features between the two inhalers 100,201 are denoted herein with like reference numerals. A principal difference between the two inhalers is in the shapes of the track parts of the cam track members 5,5' and in the shapes of the actuators 4,4'. The complimentary shapes of the cam track member 5' and actuator 4' of the second inhalation device 201 is such that (i) the first (forward) cam member 180' abuts the cam track member 5' only to drive the cam track member 5' and piercing pins 7 into the extended position, and (ii) the second (rear) cam member 190' abuts the cam track member 5' only to drive the cam track member 5' and piercing pins 7 into the retracted position. This arrangement has been found to reduce the risk of the cam track member twisting and becoming resistant to sliding with the required linear motion.

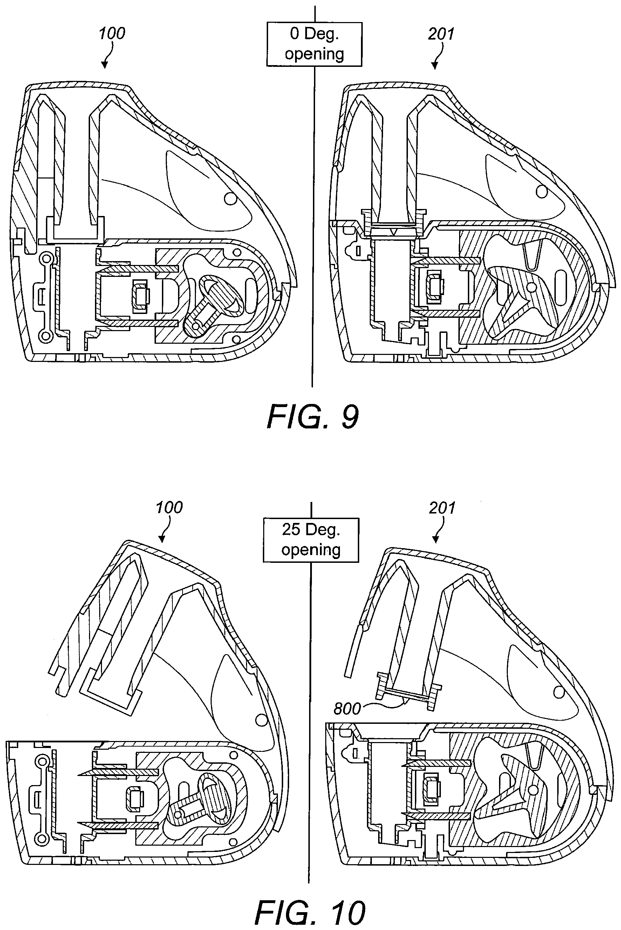

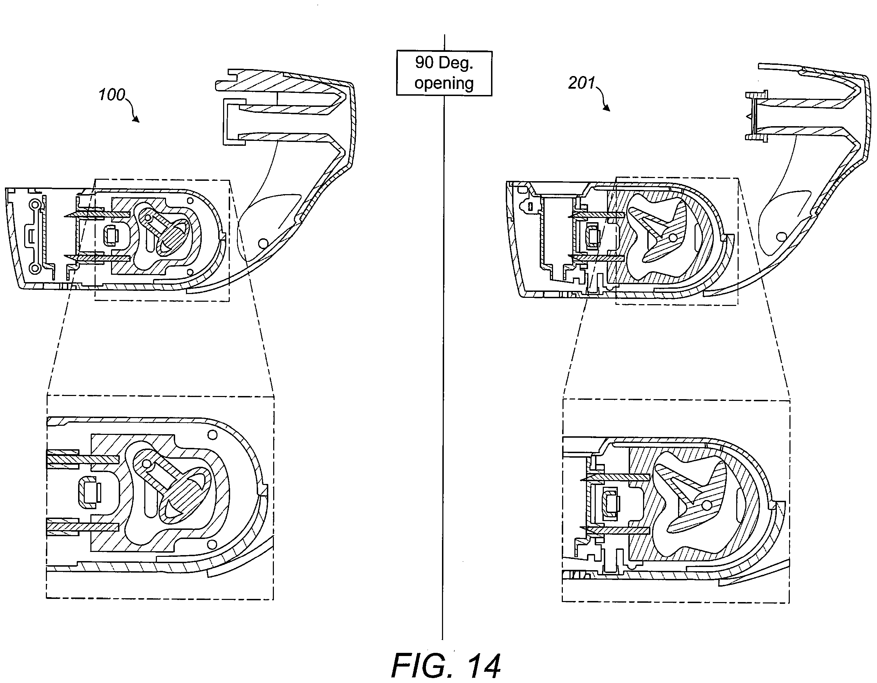

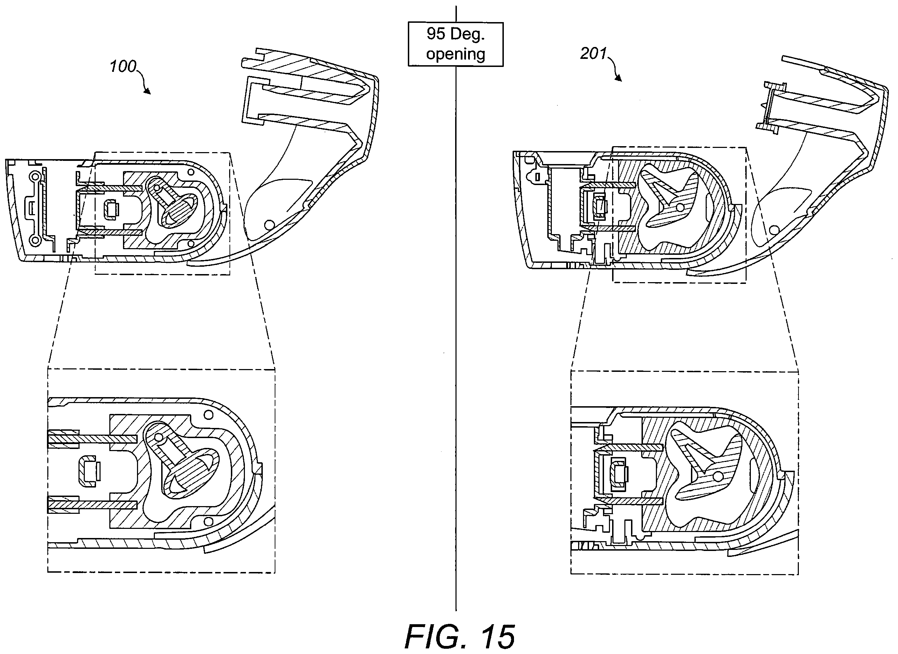

A comparison of the movement of the second inhalation device 201 with an inhalation device substantial identical with the device 100 of Figures 1 to 6 is provided in Figures 9 to 18.

In the following description, the cam track member 5' is considered to comprise four cam track parts - a first track part 200', a second track part 330', a third track part 300', and a fourth track part 230'.

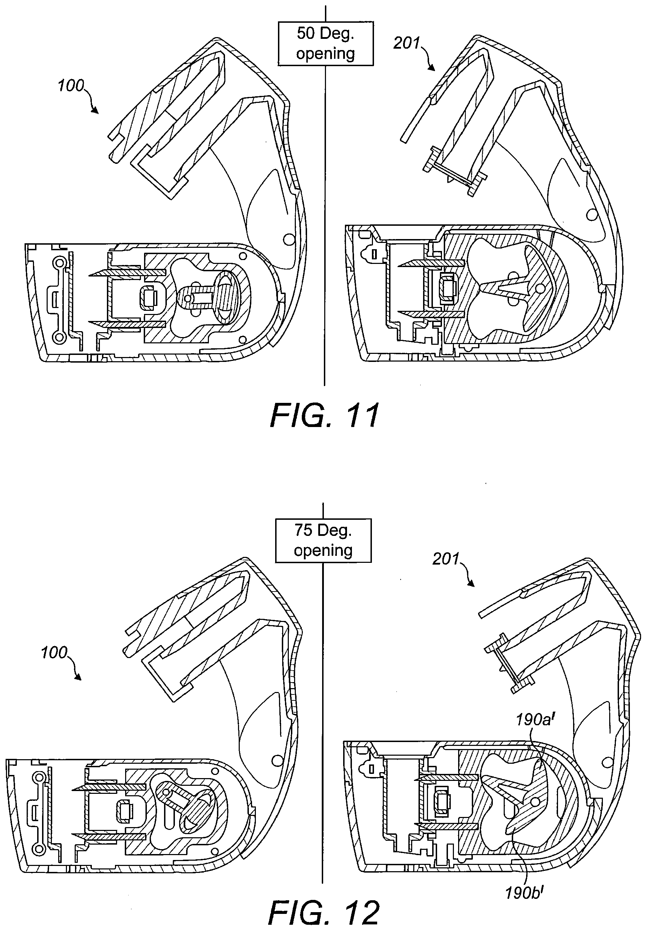

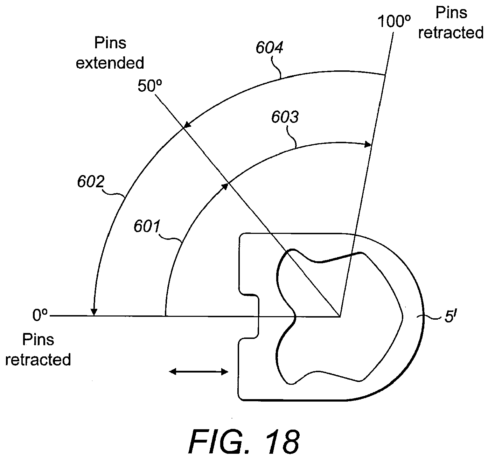

The first track part 200' is that part of the cam track located between broken lines 161 and 160a shown in Figure 8. As the mouthpiece 3 moves from the fully closed position to a position midway between the closed and opened positions, the actuator 4' is moved from the fully closed position shown in Figures 8 and 9 as a result, so that the first cam member 180' abuts and presses against the first track part 200', linearly displacing/moving the piercing cam track member 5' in a first direction. As a consequence, the piercing pins 7 are moved from the retracted position to the extended position, wherein the actuator 4' is moved through the position shown in Figure 10 to the position shown in Figure 11. The second cam member 190' does not abut the cam track member 5' during this phase of movement. It will be appreciated that as the actuator 4' moves in a rotary fashion and the track member 5' is constrained to move in a linear fashion and, as such, during the above phase of movement, the actuator 4' moves along the first track part 200' in sliding abutment therewith. In the above phase of movement, the mouthpiece 3 rotates through an arc of 50 degrees in a first movement 601 of the mouthpiece 3 shown in Figure 18.

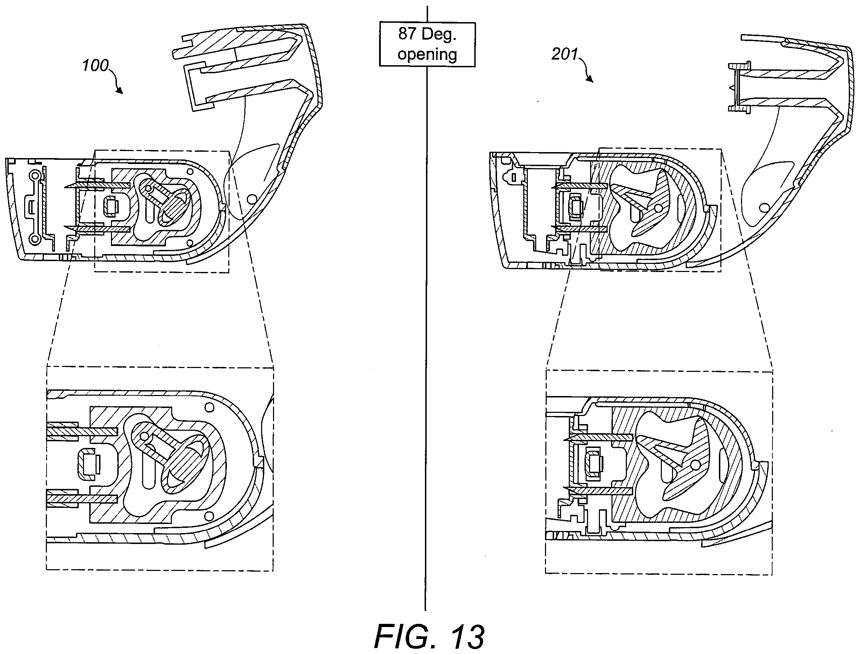

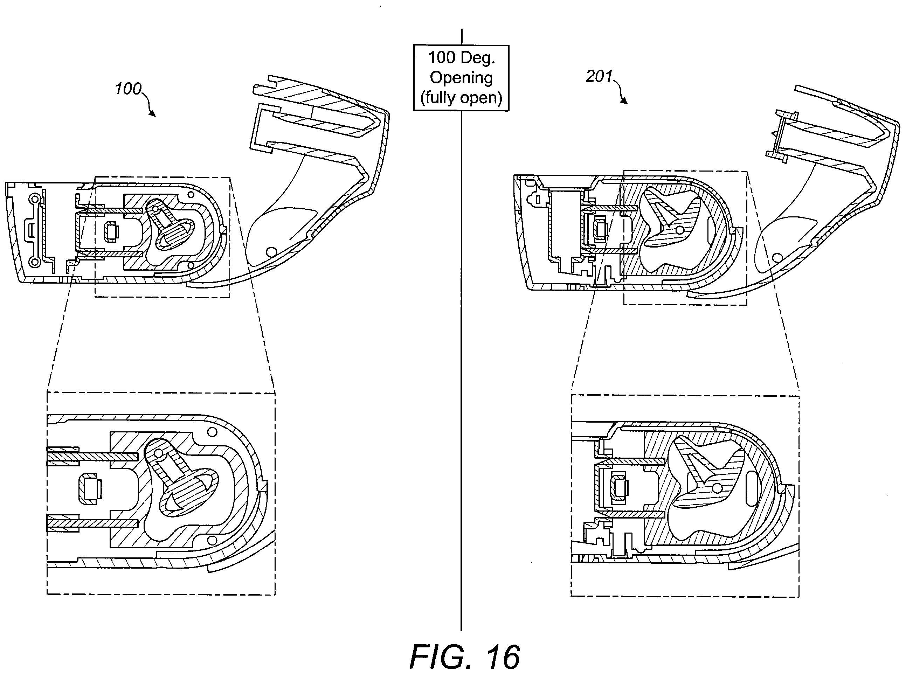

The third track part 300' is that part of the cam track located between broken line 160b and point 164 shown in Figure 8. As the mouthpiece 3 moves towards the fully opened position from a position midway between the closed and opened positions, the actuator 4' is moved from the position shown in Figure 11 as a result, so that the second cam member 190' (specifically, a first sub cam member 190a') abuts and presses against the third track part 300', linearly displacing/moving the piercing cam track member 5' in an opposite direction to said first direction. As a consequence, the piercing pins 7 are moved from the extended position all the way to the retracted position, wherein the actuator 4' is moved through the positions shown in Figures 12 to 15 (including that of Figure 13 wherein, for the equivalent position in the first inhaler, the first cam member begins to drive the retraction of the piercing pins) in to the position shown in Figure 16. In the above phase of movement, the mouthpiece 3 rotates through an arc of 50 degrees in a third movement 603 of the mouthpiece 3 shown in Figure 18.

The actuator 4' moves in a rotary fashion, and the track member 5' is constrained to move in a linear fashion. As such, during the above phases of movement, the actuator 4' moves along the third track part 300' in sliding abutment therewith.

The fourth track part 230' is that part of the cam track located between broken lines 162 and 160a shown in Figure 8. As the mouthpiece 3 moves from the fully opened position to a position midway between the closed and opened positions, the actuator 4' is moved from the position shown in Figure 16 as a result, so that the first cam member 180' abuts and presses against the fourth track part 230', linearly displacing/moving the piercing cam track member 5' in the first direction. As a consequence, the piercing pins 7 are moved from the retracted position to the extended position, wherein the actuator 4' is moved to the position shown in Figure 11. The second cam member 190' does not abut the cam track member 5' during this phase of movement. It will be appreciated that as the actuator 4' moves in a rotary fashion and the track member 5' is constrained to move in a linear fashion and, as such, during the above phase of movement, the actuator 4' moves along the fourth track part 230' in sliding abutment therewith. In the above phase of movement, the mouthpiece 3 rotates through an arc of 50 degrees in a fourth movement 604 of the mouthpiece 3 shown in Figure 18.

The second track part 330' is that part of the cam track located between broken line 160b and point 163 shown in Figure 8. As the mouthpiece 3 moves towards the fully closed position from a position midway between the closed and opened positions, the actuator 4' is moved from the position shown in Figure 11 as a result, so that the second cam member 190' (specifically, a second sub cam member 190b') abuts and presses against the second track part 330', linearly displacing/moving the piercing cam track member 5' in an opposite direction to said first direction. As a consequence, the piercing pins 7 are moved from the extended position all the way to the retracted position, wherein the actuator 4' is moved to the position shown in Figure 9. In the above phase of movement, the mouthpiece 3 rotates through an arc of 50 degrees in a second movement 602 of the mouthpiece 3 shown in Figure 18.

It will be appreciated that as the actuator 4' moves in a rotary fashion and the track member 5' is constrained to move in a linear fashion and, as such, during the above phases of movement, the actuator 4' moves along the second track part 330' in sliding abutment therewith.

Furthermore, In the second inhalation device 201, the inlet 71' is T-shaped wherein the upright of the T-shape is a first discrete aperture and the crossbar of the T-shape is a second discrete aperture, material 521 of the end shell piece separating the two discrete apertures (see Figure 8).

Also, in the second inhalation device 201, the first case member 120 is integrally formed with a third ring piece 61c and the second case member 121 is integrally formed with the fourth ring piece (not shown). In the assembled actuator case, the third ring piece 61c and the fourth ring piece locate adjacent one another to form a two-piece locating ring which is cantilevered from the remainder of the actuator case and is positioned about the boss of the chamber 14 inlet.

In this way, means are provided for supporting the lower end of the chamber 14. More specifically, the supporting means limits or prevents lateral movement of the chamber 14 at the lower end of the chamber 14, by keeping the lower end captive. The resulting reduction in movement assists in ensuring the two piercing pins 7 remain aligned with the guide means 20 and do not tend to press against the guide means 20 in a way that hinders their movement.

The mesh unit 9 includes an element 800 extending downwardly therefrom into the chamber 14. The element 800 has a conical shape with the apex thereof located lowermost within the chamber 14. A means is thereby provided for presenting a point contact for abutment with a medicament capsule in the chamber 14. In use, when a user inhales through the mouthpiece 3, the flow of inhalation air through the chamber 14 can tend to lift the medicament capsule and move said capsule upwards in the chamber 14 towards the mesh 3, which effectively closes the chamber 14 so as to retain the capsule captive in the chamber 14. If not for said point contact means, the capsule tends to press against the mesh and adhere to the mesh. However, due to the small surface area presented for abutment with the capsule, the point contact means reduces the tendency for the capsule to become adhered to the mesh.

The present invention is not limited to the specific embodiments described. Alternative arrangements and suitable materials will be apparent to a reader skilled in the art.

For example, in an alternative embodiment (not shown), the inhalation device 100 could be modified by providing a less complex actuator 4. Specifically, the actuator 4 can be modified so as to remove the second (rear) cam member 190 to leave only the first (forward) cam member 180. Whilst the design of the actuator 4 will then be relatively simple, the movement on the cam track member 5 will be less smooth and there is an increased risk of the cam track member 5 twisting and becoming resistant to sliding with the required linear motion. The piercing pins 7 would nevertheless move from the retracted position to the extended position and then back to the retracted position as the mouthpiece is moved from the closed position to the opened position. The piercing pins 7 would also move from the retracted position to the extended position and then back to the retracted position as the mouthpiece is moved from the opened position to the closed position.

The cam track member 5 of the first inhalation device 100 may be modified so that the first cam member 180 cannot abut the fifth track part 500 to return the piercing pins 7 to the retracted position. In this embodiment, only the first, second and third movements 501,502,503 of the mouthpiece 3 drive movement of the piercing pins 7, i.e. when moving the mouthpiece between zero degrees and fifty degrees. The fourth, fifth and sixth movements 504,505,506 of the mouthpiece 3 do not then drive any movement of the piercing pins 7. Accordingly, in such an embodiment, at some position of the mouthpiece between zero degrees and fifty degrees, the extension of the piercing pins 7 and the opening of the chamber 14 must be such that a medicament capsule may be positioned in the chamber 14 prior to being pierced upon further movement of the mouthpiece towards the fifty degree position.

In a further alternative embodiment (not shown), the chamber 14 comprises turbulence generating means, for example projections, on the interior wall of the chamber 14. The projections have a dual function: they both hold a capsule received within the chamber 14, and generate turbulence in fluid flow through the chamber 14 and around a capsule received therein. The arrangement (relative sizing of the projections/chamber and the capsule) is such that, when a capsule is received in the chamber 14, the projections loosely hold the capsule within the chamber 14. During use of the alternative embodiment, as air is inhaled by the user from the chamber 14 through the outlet 12 and mouthpiece 3, the projections generate turbulence in the air flow within the chamber 14. Turbulence in the air flowing around the capsule causes vibration of the capsule within the chamber 14, and this vibration enhances the dispersion of medicament contained within the capsule. Accordingly, less forceful inhalation by the user is required to liberate a full dose of medicament from the capsule.

The projections may be of various shapes, such as grooves, ridges, helixes, rings or spheres, or any other shape suitable for generating turbulence in a fluid flow.

## Claims

1. An inhaler device (100) for facilitating the inhalation of a medicament from a pierceable medicament capsule, the inhaler device comprising a body (1) having a chamber (14) for receiving a pierceable medicament capsule; piercing means (7) for piercing a medicament capsule received in said chamber (14); an actuator (4,4') rotatably mounted to the body (1); and an actuating member (3) moveable relative to the body (1) so as to rotate said actuator (4,4'); **characterised by** a cam track member (5,5') from which the piercing means (7) extend and comprising a plurality of cam track parts (200,600,200',330') along which said actuator (4,4') slides; wherein a first movement (501,601) of the actuating member (3) relative to the body (1) slides said actuator (4,4') along and in abutment with a first one (200,200') of said cam track parts so as to press against said cam track member (5,5') and thereby drive the piercing means (7) towards an extended position; and wherein a second movement (502,602) of the actuating member (3) relative to the body (1) slides said actuator (4,4') along and in abutment with a second one (600,330') of said cam track parts so as to press against said cam track member (5,5') and thereby drive the piercing means (7) towards a retracted position.

2. An inhaler device as claimed in claim 1, wherein said abutment with said first cam track part (200') is by a first cam member (180') of said actuator (4'), and said abutment with said second cam track part (330') is by a second cam member (190') of said actuator (4').

3. An inhaler device as claimed in claim 2, wherein a third movement (603) of the actuating member (3) relative to the body (1) slides said second cam member (190') of said actuator (4') along and in abutment with a third one (300') of said cam track parts so as to press against said cam track member (5') and thereby drive the piercing means (7) towards a retracted position; and wherein a fourth movement (604) of the actuating member (3) relative to the body (1) slides said first cam member (180') of said actuator (4') along and in abutment with a fourth one (230') of said cam track parts so as to press against said cam track member (5') and thereby drive the piercing means (7) towards the extended position.

4. An inhaler device as claimed in claim 3, wherein said actuating member (3) is moveable back and forth between first and second limits of movement, and wherein, movement of said actuating member (3) from the first limit to the second limit comprises in sequence said first movement (601) followed by said third movement (603) of said actuating member (3), and wherein, movement of said actuating member (3) from the second limit to the first limit comprises in sequence said fourth movement (604) followed by said second movement (602) of said actuating member (3).

5. An inhaler device as claimed in claim 1, wherein said abutment with said first cam track part (200) is by a first cam member (180) of said actuator (4), and said abutment with said second cam track part (600) is by said first cam member (180).

6. An inhaler device as claimed in claim 5, wherein said actuator (4) comprises a second cam member (190); and wherein a third movement (503) of the actuating member (3) relative to the body (1) slides said second cam member (190) along and in abutment with a third one (330) of said cam track parts so as to press against said cam track member (5) and drive the piercing means (7) from an extended position to a partially retracted position, and wherein said second movement (502) of the actuating member (3) relative to the body (1) slides said first cam member (180) of said actuator (4) along and in abutment with said second one (600) of said cam track parts so as to press against said cam track member (5) and thereby drive the piercing means (7) from said partially retracted position to a fully retracted position.

7. An inhaler device as claimed in claim 6, wherein a fourth movement (504) of the actuating member (3) relative to the body (1) slides said first cam member (180) of said actuator (4) along and in abutment with a fourth one (230) of said cam track parts so as to press against said cam track member (5) and thereby drive the piercing means (7) towards an extended position; and wherein a fifth movement (505) of the actuating member (3) relative to the body (1) slides said first cam member (180) of said actuator (4) along and in abutment with a fifth one (500) of said cam track parts so as to press against said cam track member (5) and thereby drive the piercing means (7) towards the fully retracted position; and wherein a sixth movement (506) of the actuating member (3) relative to the body (1) slides said second cam member (190) along and in abutment with a sixth one (300) of said cam track parts so as to press against said cam track member (5) and drive the piercing means (7) from an extended position to said partially retracted position, and wherein said fifth movement (505) of the actuating member (3) drives the piercing means (7) from said partially retracted position to a fully retracted position, optionally wherein said actuating member (3) is moveable back and forth between first and second limits of movement, and wherein, movement of said actuating member (3) from the first limit to the second limit comprises in sequence said first movement (501) followed by said sixth movement (506) followed by said fifth movement (505) of said actuating member (3), and wherein, movement of said actuating member (3) from the second limit to the first limit comprises in sequence said fourth movement (504) followed by said third movement (503) followed by said second movement (502) of said actuating member (3).

8. An inhaler device as claimed in claim 4 or 7, wherein, at said first limit of movement, the chamber (14) is closed by said actuating member (3) so as to retain captive in the chamber (14) a medicament capsule received in said chamber (14), and wherein, at said second limit of movement, the chamber (14) is open so as to allow release from the chamber (14) a medicament capsule received in said chamber (14).

9. An inhaler device as claimed in any of the preceding claims, wherein said body (1) comprises means constraining movement of said cam track member (5,5') to reciprocal linear movement within a single plane relative to the body (1), optionally wherein said plurality of cam track parts (200,600,200',330') are located in said single plane.

10. An inhaler device as claimed claim 9, wherein said actuator (4) is rotatable about an axis extending perpendicularly to said single plane, optionally wherein the or each cam member (180) of said actuator is located in said single plane.

11. An inhaler device as claimed claims 9 or 10, wherein:
a) the piercing means (7) is located in said single plane; and/or
b) said constraining means abuts said cam track member (5,5') so as to constrain movement thereof.

12. An inhaler device as claimed in any of the preceding claims, wherein said actuating member (3) comprises a mouthpiece (72) through which, in use, a medicament from a pierceable medicament capsule in the chamber (14) is inhaled.

13. An inhaler device as claimed in any of the preceding claims, wherein in the extended position, at least a part of the piercing means (7) extends within said chamber (14) from one side of said chamber (14) towards an opposite side thereof.

14. An inhaler device as claimed in any of the preceding claims, wherein means (800) of providing a point contact to a medicament capsule is located in the chamber (14).

15. An inhaler device as claimed in any of the preceding claims, further comprising means for supporting a lower end of the chamber (14), preferably wherein the supporting means limits or prevents lateral movement of the chamber (14) at the lower end of the chamber (14).

## Patentansprüche

1. Inhaliergerät (100) zum Ermöglichen des Inhalierens eines Medikaments aus einer durchstechbaren Medikamentenkapsel, wobei das Inhaliergerät ein Gehäuse (1) mit einer Kammer (14) zum Aufnehmen einer durchstechbaren Medikamentenkapsel; ein Durchstechmittel (7) zum Durchstechen einer in der Kammer (14) aufgenommenen Medikamentenkapsel; ein Stellglied (4,4'), das drehbar an dem Gehäuse (1) befestigt ist; und ein Betätigungselement (3), das beweglich relativ zu dem Gehäuse (1) angeordnet ist, um das Stellglied (4,4') zu drehen, umfasst; **gekennzeichnet durch** ein Nockenspurelement (5,5'), von dem sich das Durchstechelement (7) erstreckt und das eine Vielzahl von Nockenspurabschnitten (200,600,200',330') umfasst, entlang denen das Stellglied (4,4') gleitet; wobei eine erste Bewegung (501, 601) des Betätigungselements (3) relativ zu dem Gehäuse (1) das Stellglied (4,4') entlang und im Anschlag mit einem ersten (200, 200') der Nockenspurabschnitte so gleitet, dass gegen das Nockenspurelement (5,5') gedrückt und dadurch das Durchstechmittel (7) in Richtung einer ausgezogenen Position gefahren wird; und wobei eine zweite Bewegung (502,602) des Betätigungselements (3) relativ zu dem Gehäuse (1) das Stellglied (4.4') entlang und in Anschlag mit einem zweiten (600,330') der Nockenspurabschnitte so gleitet, dass gegen das Nockenspurelement (5,5') gedrückt und dadurch das Durchstechmittel (7) in Richtung einer eingefahrenen Position gefahren wird.

2. Inhaliergerät nach Anspruch 1, wobei der Anschlag mit dem ersten Nockenspurabschnitt (200') durch ein erstes Nockenelement (180') des Stellglieds (4'), und der Anschlag mit dem zweiten Nockenspurabschnitt (330') durch ein zweites Nockenelement (190') des Stellglieds (4') bewirkt wird.

3. Inhaliergerät nach Anspruch 2, wobei eine dritte Bewegung (603) des Betätigungselements (3) relativ zu dem Gehäuse (1) das zweite Nockenelement (190') des Stellglieds (4') entlang und im Anschlag mit einem dritten (300') der Nockenspurabschnitte so gleitet, dass gegen das Nockenspurelement (5') gedrückt und dadurch das Durchstechmittel (7) in Richtung einer eingezogenen Position gefahren wird; und wobei eine vierte Bewegung (604) des Betätigungselements (3) relativ zu dem Gehäuse (1) das erste Nockenelement (180') des Stellglieds (4') entlang und im Anschlag mit einem vierten (230') der Nockenspurabschnitte so gleitet, dass gegen das Nockenspurelement (5') gedrückt und dadurch das Durchstechmittel (7) in Richtung der ausgezogenen Position gefahren wird.

4. Inhaliergerät nach Anspruch 3, wobei das Betätigungselement (3) zwischen einer ersten und einer zweiten Bewegungsgrenze vor und zurück beweglich ist, und wobei die Bewegung des Betätigungselements (3) von der ersten Grenze zu der zweiten Grenze sequenziell die erste Bewegung (601), gefolgt von der dritten Bewegung (603) des Betätigungselements (3) umfasst, und wobei die Bewegung des Betätigungselements (3) von der zweiten Begrenzung zu der ersten Begrenzung sequenziell die vierte Bewegung (604), gefolgt von der zweiten Bewegung (602) des Betätigungselements (3) umfasst.

5. Inhaliergerät nach Anspruch 1, wobei der Anschlag mit dem ersten Nockenspurabschnitt (200) durch ein erstes Nockenelement (180) des Stellglieds (4) und der Anschlag mit dem zweiten Nockenspurabschnitt (600) durch das erste Nockenelement (180) bewirkt wird.

6. Inhaliergerät nach Anspruch 5, wobei das Stellglied (4) ein zweites Nockenelement (190) umfasst; und wobei eine dritte Bewegung (503) des Betätigungselements (3) relativ zu dem Gehäuse (1) das zweite Nockenelement (190) entlang und in Anschlag mit einem dritten (330) der Nockenspurabschnitte so gleitet, dass gegen das Nockenspurelement (5) gedrückt und das Durchstechmittel (7) von einer ausgezogenen Position zu einer teilweise eingezogenen Position gefahren wird, und wobei die zweite Bewegung (502) des Betätigungselements (3) relativ zu dem Gehäuse (1) ein erstes Nockenelement (180) des Stellglieds (4) entlang und in Anschlag mit einem zweiten (600) der Nockenspurabschnitte so gleitet, dass gegen das Nockenspurelement (5) gedrückt und dadurch das Durchstechmittel (7) von der teilweise eingezogenen Position zu einer vollständig eingezogenen Position gefahren wird.

7. Inhaliergerät nach Anspruch 6, wobei eine vierte Bewegung (504) des Betätigungselements (3) relativ zu dem Gehäuse (1) das erste Nockenelement (180) des Stellglieds (4) entlang und in Anschlag mit einem vierten (230) der Nockenspurabschnitte so gleitet, dass gegen das Nockenspurelement (5) gedrückt und dadurch das Durchstechmittel (7) in Richtung einer ausgezogenen Position gefahren wird; und wobei eine fünfte Bewegung (505) des Betätigungselements (3) relativ zu dem Gehäuse (1) das erste Nockenelement (180) des Stellglieds (4) entlang und in Anschlag mit einem fünften (500) der Nockenspurabschnitte so gleitet, dass gegen das Nockenspurelement (5) gedrückt und dadurch das Durchstechmittel (7) in Richtung der vollständig eingezogenen Position gefahren wird; und wobei eine sechste Bewegung (506) des Betätigungselements (3) relativ zu dem Gehäuse (1) das zweite Nockenelement (190) entlang und in Anschlag mit einem sechsten (300) der Nockenspurabschnitte so gleitet, dass gegen das Nockenspurelement (5) gedrückt und das Durchstechmittel (7) von einer ausgezogenen Position zu der teilweise eingezogenen Position gefahren wird; und wobei die fünfte Bewegung (505) des Betätigungselements (3) das Durchstechmittel (7) von der teilweise eingezogenen Position zu einer vollständig eingezogenen Position fährt, wobei optional das Betätigungselement (3) zwischen einer ersten und einer zweiten Bewegungsbegrenzung vor und zurück beweglich ist, und wobei die Bewegung des Betätigungselements (3) von der ersten Begrenzung zu der zweiten Begrenzung sequenziell die erste Bewegung (501), gefolgt von der sechsten Bewegung (506), gefolgt von der fünften Bewegung (505) des Betätigungselements (3) umfasst, und wobei die Bewegung des Betätigungselements (3) von der zweiten Begrenzung zu der ersten Begrenzung sequenziell die vierte Bewegung (504), gefolgt von der dritten Bewegung (503), gefolgt von der zweiten Bewegung (502) des Betätigungselements (3) umfasst.

8. Inhaliergerät nach Anspruch 4 oder 7, wobei an der ersten Bewegungsbegrenzung die Kammer (14) durch das Betätigungselement (3) so geschlossen wird, dass eine in der Kammer (14) aufgenommene Medikamentenkapsel in der Kammer (14) gehalten wird, und wobei an der zweiten Bewegungsbegrenzung die Kammer (14) so geöffnet wird, dass eine in der Kammer (14) aufgenommene Medikamentenkapsel von der Kammer (14) ausgegeben werden kann.

9. Inhaliergerät nach einem der vorhergehenden Ansprüche, wobei das Gehäuse (1) ein Mittel zum Einschränken der Bewegung des Nockenspurelements (5,5') auf eine reziproke lineare Bewegung in einer einzigen Ebene relativ zu dem Gehäuse (1) umfasst, wobei optional die Vielzahl von Nockenspurabschnitten (200,600,200',330') auf der einzigen Ebene vorhanden sind.

10. Inhaliergerät nach Anspruch 9, wobei das Stellglied (4) um eine Achse drehbar ist, die sich senkrecht zu der einzigen Ebene erstreckt, wobei optional das oder jedes Nockenelement (180) des Stellglieds auf der einzigen Ebene vorhanden ist.

11. Inhaliergerät nach Anspruch 9 oder 10, wobei:
a) das Durchstechmittel (7) auf der einzigen Ebene vorhanden ist und/oder
b) das Beschränkungsmittel so an das Nockenspurelement (5,5') anschlägt, dass die Bewegung desselben eingeschränkt wird.

12. Inhaliergerät nach einem der vorhergehenden Ansprüche, wobei das Betätigungselement (3) ein Mundstück (72) umfasst, durch das bei der Verwendung ein Medikament von einer durchstechbaren Medikamentenkapsel in der Kammer (14) inhaliert wird.

13. Inhaliergerät nach einem der vorhergehenden Ansprüche, wobei sich in der ausgezogenen Position wenigstens ein Teil des Durchstechmittels (7) in der Kammer (14) von einer Seite der Kammer (14) in Richtung einer gegenüberliegenden Seite davon erstreckt.

14. Inhaliergerät nach einem der vorhergehenden Ansprüche, wobei das Mittel (800) zum Bereitstellen eines Punktkontaktes zu einer Medikamentenkapsel in der Kammer (14) vorhanden ist.

15. Inhaliergerät nach einem der vorhergehenden Ansprüche, ferner umfassend ein Mittel zum Tragen eines unteren Endes der Kammer (14), wobei das tragende Mittel vorzugsweise eine seitliche Bewegung der Kammer (14) an dem unteren Ende der Kammer (14) verhindert oder beschränkt.

## Revendications

1. Dispositif inhalateur (100) pour faciliter l'inhalation d'un médicament à partir d'une capsule perçable de médicament, le dispositif inhalateur comprenant un corps (1) ayant une chambre (14) pour la réception d'une capsule perçable de médicament ; des moyens de perforation (7) pour perforer une capsule de médicament reçue dans ladite chambre (14) ; un actionneur (4,4') monté de manière pivotante sur le corps (1) ; et un élément d'actionnement (3) mobile par rapport au corps (1) afin de faire pivoter ledit actionneur (4,4') ; **caractérisé par** un élément de rampe de guidage (5,5') à partir duquel les moyens de perforation (7) s'étendent et comprenant une pluralité de parties de rampe de guidage (200,600,200',330') le long desquelles ledit actionneur (4,4') coulisse ; dans lequel un premier mouvement (501,601) de l'élément d'actionnement (3) par rapport au corps (1) coulisse ledit actionneur (4,4') le long d'une première partie et en butée contre celle-ci (200,200') desdites parties de rampe de guidage de manière à exercer une pression contre ledit élément de rampe de guidage (5,5') et à commander ainsi les moyens de perforation (7) vers une position étendue ; et dans lequel un deuxième mouvement (502,602) de l'élément d'actionnement (3) par rapport au corps (1) coulisse ledit actionneur (4,4') le long d'une deuxième partie et en butée contre celle-ci (600,330') desdites parties de rampe de guidage de manière à exercer une pression contre ledit élément de rampe de guidage (5,5') et à commander ainsi les moyens de perforation (7) vers une position rétractée.

2. Dispositif inhalateur selon la revendication 1, dans lequel ledit point d'appui avec une première partie de rampe de guidage (200') est réalisé par un premier élément à came (180') dudit actionneur (4'), et ledit point d'appui avec ladite deuxième partie de rampe de guidage (330') est réalisé par un deuxième élément à came (190') dudit actionneur (4').

3. Dispositif inhalateur selon la revendication 2, dans lequel un troisième mouvement (603) de l'élément d'actionnement (3) par rapport au corps (1) coulisse ledit deuxième élément à came (190') dudit actionneur (4') le long d'une troisième partie et en butée contre celle-ci (300') desdites parties de rampe de guidage de manière à exercer une pression contre ledit élément de rampe de guidage (5') et à commander ainsi les moyens de perforation (7) vers une position rétractée ; et dans lequel un quatrième mouvement (604) de l'élément d'actionnement (3) par rapport au corps (1) coulisse ledit premier élément à came (180') dudit actionneur (4') le long d'une quatrième partie et en butée contre celle-ci (230') desdites parties de rampe de guidage de manière à exercer une pression contre ledit élément de rampe de guidage (5') et à commander ainsi les moyens de perforation (7) vers la position étendue.

4. Dispositif inhalateur selon la revendication 3, dans lequel ledit élément d'actionnement (3) est mobile vers l'avant et vers l'arrière entre les première et deuxième limites de mouvement, et dans lequel, le mouvement dudit élément d'actionnement (3) depuis la première limite vers la deuxième limite comprend en série ledit premier mouvement (601) suivi par ledit troisième mouvement (603) dudit élément d'actionnement (3), et dans lequel, le mouvement dudit élément d'actionnement (3) à partir de la deuxième limite vers la première limite comprend en série ledit quatrième mouvement (604) suivi par ledit deuxième mouvement (602) dudit élément d'actionnement (3).

5. Dispositif inhalateur selon la revendication 1, dans lequel ledit point d'appui avec ladite première partie de rampe de guidage (200) est réalisé par un premier élément à came (180) dudit actionneur (4), et ledit point d'appui avec ladite deuxième partie de rampe de guidage (600) est réalisé par ledit premier élément à came (180).

6. Dispositif inhalateur selon la revendication 5, dans lequel ledit actionneur (4) comprend un deuxième élément à came (190) ; et dans lequel un troisième mouvement (503) de l'élément d'actionnement (3) par rapport au corps (1) coulisse ledit deuxième élément à came (190) le long d'une troisième partie et en butée contre celle-ci (330) desdites parties de rampe de guidage de manière à exercer une pression contre ledit élément de rampe de guidage (5) et à commander les moyens de perforation (7) depuis une position étendue vers une position partiellement rétractée, et dans lequel ledit deuxième mouvement (502) de l'élément d'actionnement (3) par rapport au corps (1) coulisse ledit premier élément à came (180) dudit actionneur (4) le long de ladite deuxième partie et en butée contre celle-ci (600) desdites parties de rampe de guidage de manière à exercer une pression contre ledit élément de rampe de guidage (5) et à commander ainsi les moyens de perforation (7) depuis ladite position partiellement rétractée vers une position totalement rétractée.

7. Dispositif inhalateur selon la revendication 6, dans lequel un quatrième mouvement (504) de l'élément d'actionnement (3) par rapport au corps (1) coulisse ledit premier élément à came (180) dudit actionneur (4) le long d'une quatrième partie et en butée contre celle-ci (230) desdites parties de rampe de guidage de manière à exercer une pression contre ledit élément de rampe de guidage (5) et à commander ainsi les moyens de perforation (7) vers une position étendue ; et dans lequel un cinquième mouvement (505) de l'élément d'actionnement (3) par rapport au corps (1) coulisse ledit premier élément à came (180) dudit actionneur (4) le long d'une cinquième partie et en butée contre celle-ci (500) desdites parties de rampe de guidage de manière à exercer une pression contre ledit élément de rampe de guidage (5) et à commander ainsi les moyens de perforation (7) vers la position totalement rétractée ; et dans lequel un sixième mouvement (506) de l'élément d'actionnement (3) par rapport au corps (1) coulisse ledit deuxième élément à came (190) le long d'une sixième partie et en butée contre celle-ci (300) desdites parties de rampe de guidage de manière à exercer une pression contre ledit élément de rampe de guidage (5) et à commander les moyens de perforation (7) depuis une position étendue vers ladite position rétractée partiellement, et dans lequel ledit cinquième mouvement (505) de l'élément d'actionnement (3) commande les moyens de perforation (7) depuis ladite position partiellement rétractée vers une position totalement rétractée, facultativement dans lequel ledit élément d'actionnement (3) est mobile vers l'avant et vers l'arrière entre les première et deuxième limites de mouvement, et dans lequel, le mouvement dudit élément d'actionnement (3) depuis la première limite vers la deuxième limite comprend en série ledit premier mouvement (501) suivi par ledit sixième mouvement (506) suivi par ledit cinquième mouvement (505) dudit élément d'actionnement (3), et dans lequel, le mouvement dudit élément d'actionnement (3) à partir de la deuxième limite vers la première limite comprend en série ledit quatrième mouvement (504) suivi par ledit troisième mouvement (503) suivi par ledit deuxième mouvement (502) dudit élément d'actionnement (3).

8. Dispositif inhalateur selon la revendication 4 ou 7, dans lequel, à ladite première limite de mouvement, la chambre (14) est fermée par ledit élément d'actionnement (3) afin de retenir détenue dans la chambre (14) une capsule de médicament reçue dans ladite chambre (14), et dans lequel, à ladite deuxième limite de mouvement, la chambre (14) est ouverte afin de permettre la libération depuis la chambre (14) d'une capsule de médicament reçue dans ladite chambre (14).

9. Dispositif inhalateur selon l'une quelconque des revendications précédentes, dans lequel ledit corps (1) comprend des moyens contraignant le mouvement dudit élément de rampe de guidage (5,5') au mouvement linéaire réciproque à l'intérieur d'un seul plan par rapport au corps (1), facultativement dans lequel ladite pluralité de parties de rampe de guidage (200, 600, 200', 330') sont situées dans ledit seul plan.

10. Dispositif inhalateur selon la revendication 9, dans lequel ledit actionneur (4) peut pivoter autour d'un axe s'étendant perpendiculairement vers ledit seul plan, facultativement dans lequel l'élément ou chaque élément à came (180) dudit actionneur se situe dans ledit seul plan.

11. Dispositif inhalateur selon les revendications 9 ou 10, dans lequel :
a) les moyens de perforation (7) se situent dans ledit seul plan ; et/ou
b) lesdits moyens de contrainte viennent en butée contre ledit élément de rampe de guidage (5,5') afin de restreindre le mouvement de celui-ci.

12. Dispositif ainhalateur selon l'une quelconque des revendications précédentes, dans lequel ledit élément d'actionnement (3) comprend un embout buccal (72) à travers lequel, lors de l'utilisation, un médicament est inhalé à partir d'une capsule perçable de médicament dans la chambre (14).

13. Dispositif inhalateur selon l'une quelconque des revendications précédentes, dans lequel en position étendue, au moins une partie des moyens de perforation (7) s'étend à l'intérieur de ladite chambre (14) depuis un côté de ladite chambre (14) vers un côté opposé de celle-ci.

14. Dispositif inhalateur selon l'une quelconque des revendications précédentes, dans lequel des moyens (800) de fourniture d'un point de contact à une capsule de médicament se situent dans la chambre (14).

15. Dispositif inhalateur selon l'une quelconque des revendications précédentes, comprenant en outre des moyens de support d'une extrémité inférieure de la chambre (14), de préférence dans lequel les moyens de support limitent ou empêchent le mouvement latéral de la chambre (14) au niveau de l'extrémité inférieure de la chambre (14).
